# EUROPEAN PATENT APPLICATION

(11) **EP 4 664 473 A1**
(43) Date of publication of application: **17.12.2025**
(21) Application number: 25180370.6
(22) Date of filing: 03.06.2025
(51) Int. Cl.: G16H 40/20, G16H 10/60

(54) **SYSTEM AND COMPUTER-IMPLEMENTED METHOD FOR DETERMINING HEALTHCARE GROUPER CODES WITH SUPPORTING CLINICAL CODES**

(30) Priority: 13.06.2024 US 202463659382 P
(71) Applicant: Solventum Intellectual Properties Company, Maplewood, MN 55144 (US)
(72) Inventor: Subotin, Michael Vladimir, Madison, 53717 (US); Bacon, David Robert, Sandy, 84092 (US); Hepworth, Christopher John, Centerville, 84014 (US)
(74) Representative: Vossius & Partner Patentanwälte Rechtsanwälte mbB

(57) **Abstract**

A system includes a non-transitory storage having stored thereon a machine learning model and instructions, that when executed by a processor, cause the processor to generate, via the machine learning model, an output data including a plurality of outputs for a plurality of grouper decision paths determined based on an electronic medical record and predetermined grouper guidelines. Each output for the respective grouper decision path includes: a plurality of symbols including a grouper code symbol, a plurality of decision symbols, and a plurality of clinical code symbols. The instructions further cause the processor to select one grouper code, select one or more clinical codes corresponding to the selected grouper code, determine confidence scores for the selected grouper code and the selected one or more clinical codes, and provide the selected grouper code and the selected one or more clinical codes to an automatic processing application and/or a user interface.

## Description

### Technical Field

The present disclosure generally relates to a system and a computer-implemented method for determining healthcare grouper codes with supporting clinical codes for an electronic medical record.

### Background

Healthcare groups, such as diagnosis-related groups (DRGs) and the enhanced ambulatory patient groups (EAPGs), provide patient classification schemes for relating the type of patients a healthcare facility treats (i.e., its case mix) to the costs incurred by the healthcare facility. The healthcare facility (and some health insurance companies) may utilize a grouper to categorize incurred costs based on the healthcare groups to determine how much to pay for an episode of care.

Conventionally, the grouper operates on logic that is largely defined in terms of clinical codes. The grouper may assign a grouper code to an electronic medical record (EMR) based on the clinical codes assigned to EMR. However, there are some limitations to this approach. For example, the grouper may fail to assign any DRG code to an EMR that does not have assigned clinical codes. Further, the grouper may fail to assign an appropriate DRG code to an EMR that has partial or inaccurate clinical codes assigned thereto.

### Summary

In a first aspect, the present disclosure provides a system for determining healthcare grouper codes with supporting clinical codes concerning an episode of care. The system includes one or more computer processors. The system further includes at least one non-transitory computer-readable storage. The at least one non-transitory computer-readable storage is communicatively coupled to the one or more computer processors and has stored thereon a machine learning model and instructions. The instructions, when executed by the one or more computer processors, cause the one or more computer processors to receive an electronic medical record associated with a patient. The instructions further cause the one or more computer processors to provide the electronic medical record to the machine learning model. The machine learning model is trained on a collection of historical electronic medical records. Each historical electronic medical record is paired with a grouper decision path corresponding to a previously assigned grouper code. The instructions further cause the one or more computer processors to determine, via the machine learning model, a plurality of grouper decision paths based on the electronic medical record and predetermined grouper guidelines. Each grouper decision path includes a plurality of decision nodes. The instructions further cause the one or more computer processors to determine, via the machine learning model, a plurality of grouper codes corresponding to the plurality of grouper decision paths. Each grouper code from the plurality of grouper codes is assigned to a corresponding grouper decision path from the plurality of grouper decision paths. The instructions further cause the one or more computer processors to determine, via the machine learning model, a plurality of clinical codes for each grouper decision path, such that each grouper decision path is supported by the respective plurality of clinical codes. Each clinical code from the plurality of clinical codes supports the decision made according to the predetermined grouper guidelines at a corresponding decision node from the plurality of decision nodes of the respective grouper decision path. Each clinical code includes a plurality of characters. The instructions further cause the one or more computer processors to generate, via the machine learning model, an output data including a plurality of outputs for the plurality of grouper decision paths. Each output for the respective grouper decision path includes a plurality of symbols and a plurality of confidence scores corresponding to the plurality of symbols. Each symbol from the plurality of symbols has a corresponding confidence score from the plurality of confidence scores. The plurality of symbols of each output includes a grouper code symbol representing the grouper code of the respective grouper decision path, such that the plurality of outputs includes a plurality of grouper code symbols having respective confidence scores. The plurality of symbols of each output further includes a plurality of decision symbols representing the decisions made at the plurality of decision nodes of the respective grouper decision path. Each decision symbol from the plurality of decision symbols represents the decision made at the corresponding decision node from the plurality of decision nodes. The plurality of symbols of each output further includes a plurality of clinical code symbols representing the plurality of clinical codes of the respective grouper decision path. Each clinical code symbol from the plurality of clinical code symbols represents at least one character from the plurality of characters of the corresponding clinical code. The instructions further cause the one or more computer processors to select one grouper code from the plurality of grouper codes based on the respective confidence scores of the plurality of grouper code symbols. The instructions further cause the one or more computer processors to select one or more clinical codes from the plurality of clinical codes of the grouper decision path corresponding to the selected grouper code based on the confidence scores of the plurality of clinical code symbols. Each clinical code from the selected one more clinical codes is at least a partial clinical code. The instructions further cause the one or more computer processors to determine if the confidence score of the grouper code symbol of the selected grouper code and respective code confidence scores of the selected one or more clinical codes exceed corresponding confidence score thresholds specified by a user. The code confidence score of each clinical code is a function of the confidence scores of the plurality of clinical code symbols of the clinical code. The instructions further cause the one or more computer processors to provide the selected grouper code and the selected one or more clinical codes to at least one of an automatic processing application and a user interface.

In a second aspect, the present disclosure provides a computer-implemented method for determining healthcare grouper codes with supporting clinical codes concerning an episode of care. The computer-implemented method includes receiving an electronic medical record associated with a patient. The computer-implemented method further includes providing the electronic medical record to a machine learning model. The machine learning model is trained on a collection of historical electronic medical records. Each historical electronic medical record is paired with a grouper decision path corresponding to a previously assigned grouper code. The computer-implemented method further includes determining, via the machine learning model, a plurality of grouper decision paths based on the electronic medical record and the predetermined grouper guidelines. Each grouper decision path includes a plurality of decision nodes. The computer-implemented method further includes determining, via the machine learning model, a plurality of grouper codes corresponding to the plurality of grouper decision paths. Each grouper code from the plurality of grouper codes is assigned to a corresponding grouper decision path from the plurality of grouper decision paths. The computer-implemented method further includes determining, via the machine learning model, a plurality of clinical codes for each grouper decision path, such that each grouper decision path is supported by the respective plurality of clinical codes. Each clinical code from the plurality of clinical codes supports the decision made according to the predetermined grouper guidelines at a corresponding decision node from the plurality of decision nodes of the respective grouper decision path. Each clinical code includes a plurality of characters. The computer-implemented method further includes generating, via the machine learning model, an output data including a plurality of outputs for the plurality of grouper decision paths. Each output for the respective grouper decision path includes a plurality of symbols and a plurality of confidence scores corresponding to the plurality of symbols. Each symbol from the plurality of symbols has a corresponding confidence score from the plurality of confidence scores. The plurality of symbols of each output includes a grouper code symbol representing the grouper code of the respective grouper decision path, such that the plurality of outputs includes a plurality of grouper code symbols having respective confidence scores. The plurality of symbols of each output further includes a plurality of decision symbols representing the decisions made at the plurality of decision nodes of the respective grouper decision path. Each decision symbol from the plurality of decision symbols represents the decision made at the corresponding decision node from the plurality of decision nodes. The plurality of symbols of each output further includes a plurality of clinical code symbols representing the plurality of clinical codes of the respective grouper decision path. Each clinical code symbol from the plurality of clinical code symbols represents at least one character from the plurality of characters of the corresponding clinical code. The computer-implemented method further includes selecting one grouper code from the plurality of grouper codes based on the respective confidence scores of the plurality of grouper code symbols. The computer-implemented method further includes selecting one or more clinical codes from the plurality of clinical codes of the grouper decision path corresponding to the selected grouper code based on the confidence scores of the plurality of clinical code symbols. Each clinical code from the selected one more clinical codes is at least a partial clinical code. The computer-implemented method further includes determining if the confidence score of the grouper code symbol of the selected grouper code and respective code confidence scores of the selected one or more clinical codes exceed the corresponding confidence score thresholds specified by a user. The code confidence score of each clinical code is a function of the confidence scores of the plurality of clinical code symbols of the clinical code. The computer-implemented method further includes providing the selected grouper code and the selected one or more clinical codes to at least one of an automatic processing application and a user interface.

The details of one or more examples of the disclosure are set forth in the accompanying drawings and the description below. Other features, objects, and advantages of the disclosure will be apparent from the description and drawings, and from the claims.

### Brief Description of the Drawings

Exemplary embodiments disclosed herein may be more completely understood in consideration of the following detailed description in connection with the following figures. The figures are not necessarily drawn to scale. Like numbers used in the figures refer to like components. However, it will be understood that the use of a number to refer to a component in a given figure is not intended to limit the component in another figure labeled with the same number.
FIG. 1 is a schematic block diagram of a system for determining healthcare grouper codes with supporting clinical codes according to an embodiment of the present disclosure;
FIG. 2 is a schematic diagram of a grouper decision tree corresponding to a healthcare group and its grouper guidelines according to an embodiment of the present disclosure;
FIG. 3 is a schematic diagram of a grouper decision path of the decision tree of FIG.2 according to an embodiment of the present disclosure;
FIG. 4 is a schematic block diagram of an output data generated by a machine learning model according to another embodiment of the present disclosure;
FIG. 5 is a schematic block diagram of the machine learning model according to an embodiment of the present disclosure; and
FIG. 6 is a flowchart depicting various steps of a computer-implemented method for determining healthcare grouper codes with supporting clinical codes according to an embodiment of the present disclosure.

### Detailed Description

In the following description, reference is made to the accompanying figures that form a part thereof and in which various embodiments are shown by way of illustration. It is to be understood that other embodiments are contemplated and may be made without departing from the scope or spirit of the present disclosure. The following detailed description, therefore, is not to be taken in a limiting sense.

In the following disclosure, the following definitions are adopted.

As recited herein, all numbers should be considered modified by the term "about." As used herein, "a," "an," "the," "at least one," and "one or more" are used interchangeably.

As used herein as a modifier to a property or attribute, the term "generally," unless otherwise specifically defined, means that the property or attribute would be readily recognizable by a person of ordinary skill but without requiring absolute precision or a perfect match (e.g., within +/- 20 % for quantifiable properties).

The term "substantially," unless otherwise specifically defined, means to a high degree of approximation (e.g., within +/- 10% for quantifiable properties) but again without requiring absolute precision or a perfect match.

The term "about," unless otherwise specifically defined, means to a high degree of approximation (e.g., within +/- 5% for quantifiable properties) but again without requiring absolute precision or a perfect match.

Terms such as same, equal, uniform, constant, strictly, and the like, are understood to be within the usual tolerances or measuring error applicable to the particular circumstance rather than requiring absolute precision or a perfect match.

As used herein, when a first material is termed as "similar" to a second material, at least 90% by weight of the first and second materials are identical and any variation between the first and second materials comprises less than about 10% by weight of each of the first and second materials.

As used herein, "at least one of A and B" and "at least one of A or B" should be understood to mean "only A, only B, or both A and B."

As used herein, the term "configured to" and like is at least as restrictive as the term "adapted to" and requires actual design intention to perform the specified function rather than mere physical capability of performing such a function.

As used herein, the terms "medical" and "clinical" are interchangeable and have the same meaning.

As used herein, the term "patient," and its equivalents, refers to an individual being monitored and/or cared for within a clinical environment or who has been previously monitored and/or cared for within the clinical environment. In various examples, a patient is a human, but implementations of this disclosure are not limited thereto. Examples of the clinical environment may include, but are not limited to, a doctor's office, a medical facility, a medical practice, a medical lab, an urgent care facility, a medical clinic, an emergency room, an operating room, a hospital, a long term care facility, a rehabilitation facility, a nursing home, and a hospice facility.

As used herein, the term "electronic medical record" or "EMR" refers to documents (e.g., clinical notes), database entries, and the like, that include clinical information of a patient and are stored in a computer-readable format. For example, EMR may include information associated with one or more of diagnoses, medicines, tests, allergies, immunizations, treatment plans, any suitable characteristics associated with the patient, any suitable conditions associated with the patient, and the like.

As used herein, the term "healthcare group" refers to a classification scheme that provides a means of relating the type of patients a healthcare facility treats (i.e., its case mix) to the costs incurred by the healthcare facility. An example of a healthcare group includes diagnosis-related group, which is based on primary (or principal) and secondary diagnoses, other conditions (comorbidities), age, sex, and necessary medical procedures. The term "grouper code" refers to a code assigned to an EMR based on the classification scheme defined by the healthcare group.

As used herein, the term "clinical code" refers to a code used to describe medical, surgical, and diagnostic services rendered to a patient. Clinical codes may include, for example, the International Classification of Diseases (ICD) (e.g., 10th revision) and the Current Procedural Terminology (CPT) published by the American Medical Association (e.g., CPT 2018). Clinical codes are typically assigned to an EMR by medical coders.

As used herein, the term "confidence score" refers to a value assigned by a machine learning model to an output of the machine learning model which represents a degree of certainty of the output. Confidence scores may be characterized using various conventions. One example includes a numerical value ranging from 0 to 1, where 0 represents no certainty and 1 represents absolute certainty.

As used herein, the term "processor" or "computer processor" refers any device that performs logic operations. A computer processor may include a general processor, a central processing unit, an application specific integrated circuit (ASIC), a digital signal processor, a field programmable gate array (FPGA), a digital circuit, an analog circuit, a controller, a microcontroller, any other type of processor, or any combination thereof.

As used herein, the term "instructions" refers to code (e.g., source code, compiled code, code that can be interpreted, executable code, etc.) that, when executed by a processor, causes the processor to perform various steps, functions, operations, and/or calculations, i.e., the conventional meaning of the term "instructions" with respect to digital technology.

As used herein, the term "storage device" refers to any storage medium that is capable of storing data and information in an electronic format. Examples of a storage device include hard drives, flash drives, optical media, and the like.

As used herein, the term "communicatively coupled" refers to any type of connection or coupling that allows for the exchange or sharing of information. Two communicatively coupled components may be electrically coupled by, for example, a wire; optically coupled by, for example, an optical cable; and/or wirelessly coupled by, for example, a radio frequency or other transmission media. Two communicatively coupled components may be directly coupled, or indirectly coupled, such as via a network.

As used herein, the term "machine learning model" or "ML model" refers to a machine learning algorithm or collection of algorithms that takes structured and/or unstructured data inputs and generates a prediction or result. That is, a machine learning model may be a computer model or a computer representation that may be tuned (e.g., trained) based on inputs to approximate unknown functions. The process of building or optimizing a machine learning model is referred to herein as "training." Examples of machine-learning models include, for example, one or more of vectorization machine-learning models, sequence-to-sequence models, transformer models, a decision tree (e.g., a gradient boosted decision tree), a linear regression model, a logistic regression model, association rule learning, inductive logic programming, support vector learning, a Bayesian network, a regression-based model, a neural network, or combinations thereof.

As used herein, the term "embedding" refers to a mathematical representation of a set of data points in a lower-dimensional space that captures their underlying relationships and patterns. Embeddings are often used to represent complex data types, such as images, text, or audio, in a way which machine learning algorithms can easily process. Embeddings may be a numerical or vector representation of a variable. One example of an embedding is a token embedding. In the context of the present disclosure, embeddings encompass additive combination of text embeddings and concept embeddings.

As used herein, the term "neural network" may refer to one example of a machine learning model that can be tuned (e.g., trained) based on inputs to approximate unknown functions. In particular, the neural network may include a model of interconnected neurons (arranged in layers) that communicate and learn to approximate complex functions and generate outputs based on a plurality of inputs provided to the model. For example, the neural network may include deep neural network (DNN), deep convolutional neural networks (CNN), Region-CNN (R-CNN), Faster R-CNN, Mask R-CNN, fully convolutional neural networks, recurrent neural networks ("RNNs"), such as long short-term memory neural networks ("LSTMs"), graph neural networks, generative adversarial neural networks (GAN), and single-shot detect (SSD) networks. In other words, a neural network is an algorithm that implements deep learning techniques, which utilize a set of learned parameters arranged in layers according to a particular architecture to attempt to model high-level abstractions in data using supervisory data to tune parameters of the neural network.

The present disclosure provides a system for determining healthcare grouper codes with supporting clinical codes concerning an episode of care. The system includes one or more computer processors. The system further includes at least one non-transitory computer-readable storage. The at least one non-transitory computer-readable storage is communicatively coupled to the one or more computer processors and has stored thereon a machine learning model and instructions. The instructions, when executed by the one or more computer processors, cause the one or more computer processors to receive an electronic medical record associated with a patient. The instructions further cause the one or more computer processors to provide the electronic medical record to the machine learning model. The machine learning model is trained on a collection of historical electronic medical records. Each historical electronic medical record is paired with a grouper decision path corresponding to a previously assigned grouper code. The instructions further cause the one or more computer processors to determine, via the machine learning model, a plurality of grouper decision paths based on the electronic medical record and predetermined grouper guidelines. Each grouper decision path includes a plurality of decision nodes. The instructions further cause the one or more computer processors to determine, via the machine learning model, a plurality of grouper codes corresponding to the plurality of grouper decision paths. Each grouper code from the plurality of grouper codes is assigned to a corresponding grouper decision path from the plurality of grouper decision paths. The instructions further cause the one or more computer processors to determine, via the machine learning model, a plurality of clinical codes for each grouper decision path, such that each grouper decision path is supported by the respective plurality of clinical codes. Each clinical code from the plurality of clinical codes supports the decision made according to the predetermined grouper guidelines at a corresponding decision node from the plurality of decision nodes of the respective grouper decision path. Each clinical code includes a plurality of characters. The instructions further cause the one or more computer processors to generate, via the machine learning model, an output data including a plurality of outputs for the plurality of grouper decision paths. Each output for the respective grouper decision path includes a plurality of symbols and a plurality of confidence scores corresponding to the plurality of symbols. Each symbol from the plurality of symbols has a corresponding confidence score from the plurality of confidence scores. The plurality of symbols of each output includes a grouper code symbol representing the grouper code of the respective grouper decision path, such that the plurality of outputs includes a plurality of grouper code symbols having respective confidence scores. The plurality of symbols of each output further includes a plurality of decision symbols representing the decisions made at the plurality of decision nodes of the respective grouper decision path. Each decision symbol from the plurality of decision symbols represents the decision made at the corresponding decision node from the plurality of decision nodes. The plurality of symbols of each output further includes a plurality of clinical code symbols representing the plurality of clinical codes of the respective grouper decision path. Each clinical code symbol from the plurality of clinical code symbols represents at least one character from the plurality of characters of the corresponding clinical code. The instructions further cause the one or more computer processors to select one grouper code from the plurality of grouper codes based on the respective confidence scores of the plurality of grouper code symbols. The instructions further cause the one or more computer processors to select one or more clinical codes from the plurality of clinical codes of the grouper decision path corresponding to the selected grouper code based on the confidence scores of the plurality of clinical code symbols. Each clinical code from the selected one more clinical codes is at least a partial clinical code. The instructions further cause the one or more computer processors to determine if the confidence score of the grouper code symbol of the selected grouper code and respective code confidence scores of the selected one or more clinical codes exceed corresponding confidence score thresholds specified by a user. The code confidence score of each clinical code is a function of the confidence scores of the plurality of clinical code symbols of the clinical code. The instructions further cause the one or more computer processors to provide the selected grouper code and the selected one or more clinical codes to at least one of an automatic processing application and a user interface.

The system of the present disclosure may automate healthcare revenue cycle while maintaining compliance with the predetermined grouper guidelines. Specifically, the system may determine appropriate grouper codes as well as compliant clinical codes (as per the predetermined grouper guidelines) based on the electronic medical record without requiring human review. In other words, the system may ensure that billed clinical codes are consistent with billed grouper codes. The system may leverage machine learning technologies to improve a speed and an accuracy of billing while reducing human resource time involved in billing.

In some examples, the automatic processing application may be a billing application. The system may thus enable direct-to-bill (D2B) workflows. For example, the system may enable D2B workflows if the selected one or more clinical codes have the respective code confidence scores that exceed the corresponding confidence score thresholds.

In some examples, where the code confidence scores of the selected one or more clinical codes do not exceed the corresponding confidence score thresholds, the system may expedite a workflow of the user, as the user may only need to verify the selected one or more clinical codes whose code confidence scores do not exceed the corresponding confidence score thresholds.

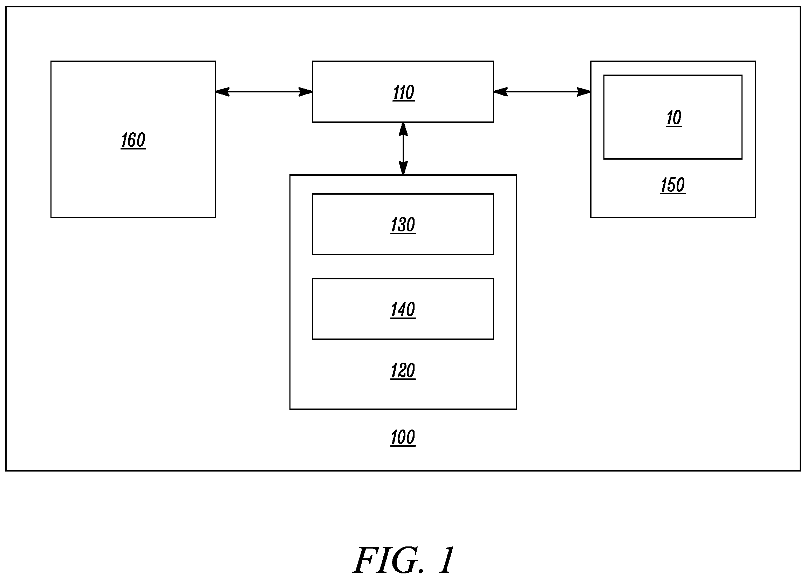

FIG. 1 illustrates a schematic block diagram of a system 100 for determining healthcare grouper codes with supporting clinical codes concerning an episode of care according to an embodiment of the present disclosure.

The system 100 includes one or more computer processors 110 (hereinafter referred to as "the processor 110"). The system 100 further includes at least one non-transitory computer-readable storage 120 (hereinafter referred to as "the non-transitory storage 120") communicatively coupled to the processor 110. The non-transitory storage 120 has stored thereon a machine learning model 130 and instructions 140. In other words, the machine learning model 130 and the instructions 140 are stored on the non-transitory storage 120.

The processor 110 may further be communicatively coupled to a storage device 150. The storage device 150 may store an electronic medical record 10 associated with a patient. The electronic medical record 10 may include structured and/or unstructured medical data related to the patient. For example, the electronic medical record 10 may include clinical notes associated with the patient. In some examples, the system 100 may include the storage device 150. Alternatively, in some other examples, the non-transitory storage 120 may store the electronic medical record 10 associated with the patient.

The processor 110 may further be communicatively coupled to an output device 160. The processor 110 may display one or more outputs to a user via the output device 160, for example, in the form of a user interface. The output device 160 may include, but is not limited to, a monitor.

As will be discussed in greater detail below, the instructions 140, when executed by the processor 110, may cause the processor 110 to receive the electronic medical record 10, provide the electronic medical record 10 to the machine learning model 130, receive an output from the machine learning model 130, and display the output to the user via the output device 160.

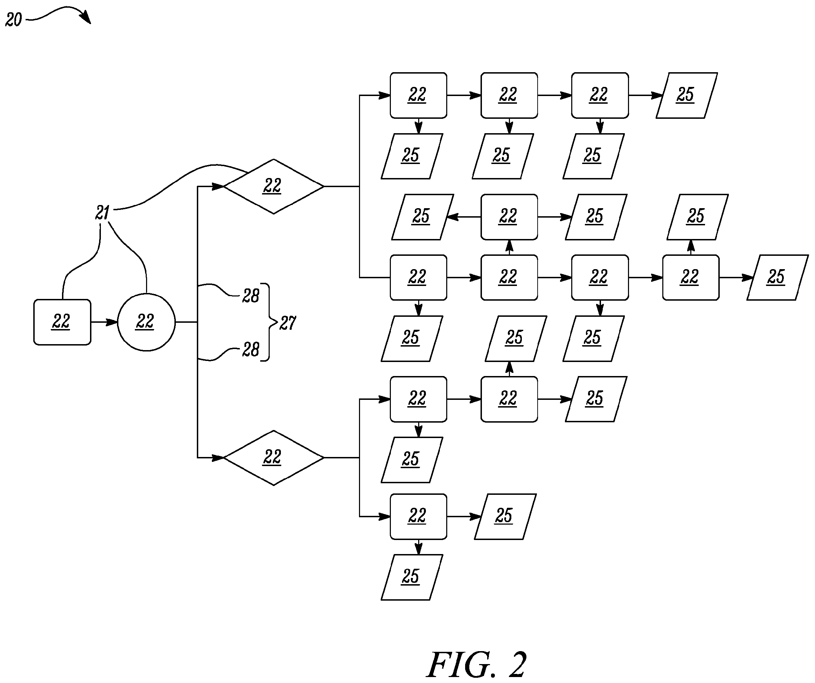

FIG. 2 illustrates a schematic diagram of a portion of a grouper decision tree 20 corresponding to a healthcare group according to an embodiment of the present disclosure. It should be noted that the grouper decision tree 20 shown in FIG. 2 is illustrative in nature and may not be representative of an actual healthcare group.

The grouper decision tree 20 may conform to guidelines of the healthcare group, and therefore may be interchangeably referred to as "the predetermined grouper guidelines 20." In some embodiments, the predetermined grouper guidelines 20 may correspond to at least one of a diagnosis-related group (DRG) and an enhanced ambulatory patient group (EAPG).

The grouper decision tree 20 may include a plurality of grouper decision paths 27. Each grouper decision path 28 from the plurality of grouper decision paths 27 may include a plurality of decision nodes 21. Each decision node 22 from the plurality of decision nodes 21 may include a decision criterion associated with a clinical diagnosis of the patient. As a non-limiting example, the plurality of decision nodes 21 may answer questions such as "what is the principal diagnosis of the clinical diagnosis?," "which major diagnostic category (MDC) does the principal diagnosis fall into?," "did the patient undergo an operating room (OR) procedure?," "did a complication or comorbidity (CC) or a major complication or comorbidity (MCC) occur during treatment of the patient?," and so forth. Different healthcare groups may have different decision criteria.

The grouper decision tree 20 further includes a plurality of grouper codes 25 corresponding to the plurality of grouper decision paths 27. Each grouper code 25 from the plurality of grouper codes 25 is assigned to a corresponding grouper decision path 28 from the plurality of grouper decision paths 27. Each grouper decision path 28 may terminate at the corresponding grouper code 25.

The grouper decision tree 20 may be traversed based on the decision made at each decision node 22 from the plurality of decision nodes 21 with respect to the medical data of the electronic medical record 10 (shown in FIG. 1). Specifically, traversing the grouper decision tree 20 based on the electronic medical record 10 may provide an appropriate grouper decision path 28 for the electronic medical record 10, and consequently, an appropriate grouper code 25 for the electronic medical record 10.

The plurality of decision nodes 21 may be arranged in a node sequence in each grouper decision path 28. The node sequence may be a tuple having a length k, defined as (D1, D2, D3, ...., Dk), where D1 and Dk are respective first and last decision nodes 22 of the grouper decision path 28.

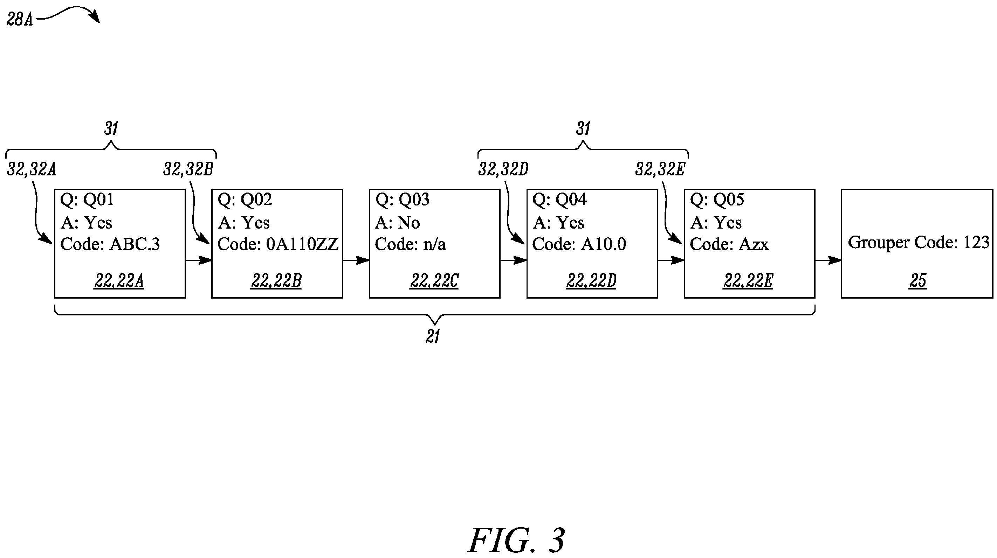

One grouper decision path 28A from the plurality of decision grouper paths 27 is depicted in isolation in FIG. 3. The grouper decision path 28A shown in FIG. 3 may be the appropriate grouper decision path 28 from the plurality of grouper decision paths 27 of FIG. 2.

Referring to FIGS. 2 and 3, the grouper decision path 28A may include the plurality of decision nodes 21. Specifically, in the illustrated example of FIG. 3, the plurality of decision nodes 21 includes decision nodes 22A, 22B, 22C, 22D, 22E. Further, in the illustrated example of FIG. 3, the node sequence of the plurality of decision nodes 21 is (22A, 22B, 22C, 22D, 22E).

The grouper decision path 28A may be associated with a plurality of clinical codes 31. Each clinical code 32 from the plurality of clinical codes 31 supports the decision according to the predetermined grouper guidelines 20 at a corresponding decision node 22 from the plurality of decision nodes 21 of the respective grouper decision path 28A. Each clinical code 32 from the plurality of clinical codes 31 includes a plurality of characters. The plurality of characters of each clinical code 32 may include alphabets, numbers, and other characters.

It may be noted that each clinical code 32 may be associated with the corresponding decision node 22. However, there may exist one or more decision nodes 22 that do not have any clinical code associated thereto (e.g., the decision node 22C in FIG. 3). In the illustrated example of FIG. 3, a clinical code 32A is assigned to the decision node 22A, a clinical code 32B is assigned to the decision node 22B, a clinical code 32D is assigned to the decision node 22D, and a clinical code 32E is assigned to the decision node 22E. Further, the grouper decision path 28A terminates at the corresponding grouper code 25.

Referring now to FIGS. 1 to 3, the instructions 140, when executed by the processor 110, cause the processor 110 to receive the electronic medical record 10 associated with the patient. For example, the processor 110 may query a database (not shown) storing the electronic medical record 10 to retrieve the electronic medical record 10 associated with the patient. The database may be stored on the storage device 150. The database may be maintained by a healthcare facility, such as a hospital.

The instructions 140 further cause the processor 110 to provide the electronic medical record 10 to the machine learning model 130. The machine learning model 130 is trained on a collection of historical electronic medical records. Each historical electronic medical record is paired with a grouper decision path (e.g., the grouper decision path 28A shown in FIG. 3) corresponding to a previously assigned grouper code. In other words, the machine learning model 130 may be trained to determine a plurality of grouper decision paths (e.g., the plurality of grouper decision paths 27 of FIG. 2) based on the electronic medical record 10 and the predetermined grouper guidelines 20. The machine learning model 130 may be trained using any suitable learning technique, such as supervised learning, unsupervised learning, semi-supervised learning, reinforcement learning, and the like.

The instructions 140 further cause the processor 110 to determine, via the machine learning model 130, the plurality of grouper decision paths 27 based on the electronic medical record 10 and the predetermined grouper guidelines 20.

The instructions 140 further cause the processor 110 to determine, via the machine learning model 130, the plurality of grouper codes 25 corresponding to the plurality of grouper decision paths 27.

The instructions 140 further cause the processor 110 to determine, via the machine learning model 130, the plurality of clinical codes 31 for each grouper decision path 28, such that each grouper decision path 28 is supported by the respective plurality of clinical codes 31.

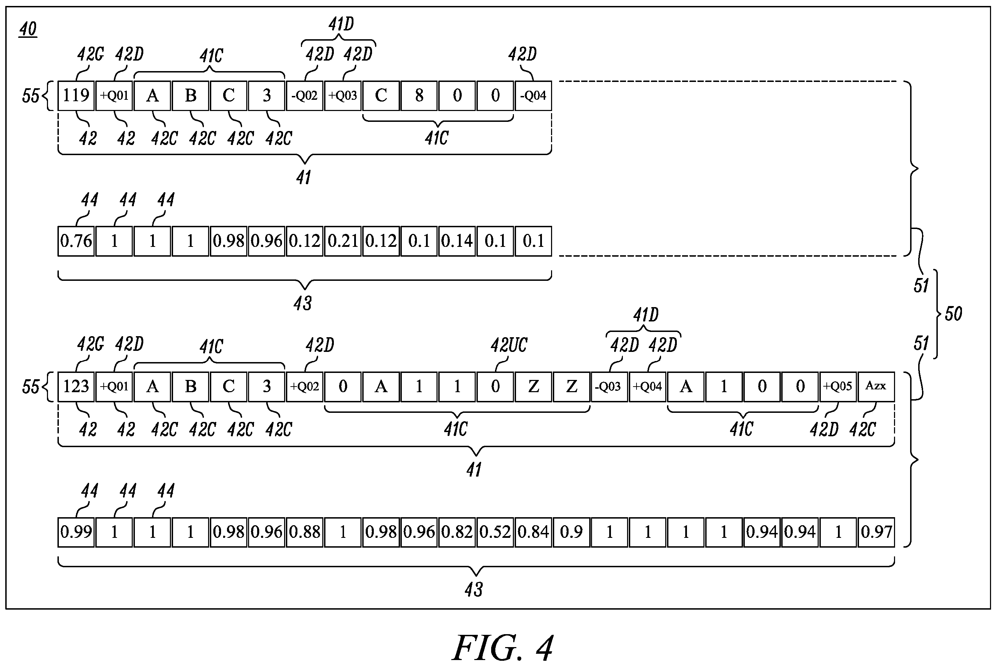

The instructions 140 further cause the processor 110 to generate, via the machine learning model 130, an output data 40 (shown in FIG. 4). For explanatory purposes, it may be assumed that the grouper decision path 28A of FIG. 3 is the appropriate grouper decision path 28 for the electronic medical record 10.

Referring now to FIGS. 1 to 4, the output data 40 includes a plurality of outputs 50 for the plurality of grouper decision paths 27. Each output 51 for the respective grouper decision path 28 includes a plurality of symbols 41 and a plurality of confidence scores 43 corresponding to the plurality of symbols 41. Each symbol 42 from the plurality of symbols 41 has a corresponding confidence score 44 from the plurality of confidence scores 43. The machine learning model 130 may determine a certainty of correctness of each symbol 42 to assign the corresponding confidence score 44 to the symbol 42.

As shown in FIG. 4, in some embodiments, each output 51 includes a vector 55 including the plurality of symbols 41. The vector 55 may correspond to a row vector or a column vector. In the illustrated embodiment of FIG. 4, the vector 55 of each output 51 is a row vector.

The plurality of symbols 41 of each output 51 includes a grouper code symbol 42G representing the grouper code 25 of the respective grouper decision path 28, such that the plurality of outputs 50 includes a plurality of grouper code symbols 42G having respective confidence scores 44.

The plurality of symbols 41 of each output 51 further includes a plurality of decision symbols 41D representing the decisions made at the plurality of decision nodes 21 of the respective grouper decision path 28. Specifically, each decision symbol 42D from the plurality of decision symbols 41D represents the decision made at the corresponding decision node 22 from the plurality of decision nodes 21.

The plurality of symbols 41 of each output 51 further includes a plurality of clinical code symbols 41C representing the plurality of clinical codes 31 of the respective grouper decision path 28.

Each clinical code symbol 42C from the plurality of clinical code symbols 41C represents at least one character from the plurality of characters of the corresponding clinical code 32. In some embodiments, for each decision node 22 of the respective grouper decision path 28, the corresponding output 51 includes a set of clinical code symbols 41C arranged in a code sequence to together form the clinical code 32 assigned to the decision made at the decision node 22. The clinical code 32 may be a concatenation of the set of clinical code symbols 41C in the code sequence.

In some embodiments, the plurality of decision symbols 41D and the plurality of clinical code symbols 41C are together arranged in an output sequence that corresponds to the node sequence of the plurality of decision nodes 21.

The grouper code symbol 42G may be arranged at a beginning or an end of the output sequence. In the illustrated embodiment of FIG. 4, the grouper code symbol 42G is arranged at the beginning of the output sequence.

In some embodiments, for each decision node 22 with an assigned clinical code 32, the corresponding one or more decision symbols 42D may be followed by the corresponding one or more clinical code symbols 42C in the output sequence. Further, for each decision node 22 of the respective grouper decision path 28 without an assigned clinical code, the corresponding output 51 may be devoid of any clinical code symbol corresponding to the decision node 22.

The instructions 140 further cause the processor 110 to select one grouper code 25 from the plurality of grouper codes 25 based on the respective confidence scores 44 of the plurality of grouper code symbols 42G. In some implementations, the grouper code 25 corresponding to the grouper code symbols 42G with the highest confidence score 44 may be selected. For example, in the output data 40 shown in FIG. 4, the grouper code "123" may be the selected grouper code 25.

The instructions 140 further cause the processor 110 to select one or more clinical codes 32 from the plurality of clinical codes 31 of the grouper decision path 28 corresponding to the selected grouper code 25 based on the confidence scores 44 of the plurality of clinical code symbols 41C. Each clinical code 32 from the selected one more clinical codes 32 is at least a partial clinical code. In other words, each clinical code 32 from the selected one more clinical codes 32 may be complete (i.e., include all characters) or incomplete (i.e., devoid of some characters).

The instructions 140 further cause the processor 110 to determine if the confidence score 44 of the grouper code symbol 42G of the selected grouper code 25 and respective code confidence scores of the selected one or more clinical codes 32 exceed corresponding confidence score thresholds specified by the user. The code confidence score of each clinical code 32 is a function of the confidence scores 44 of the plurality of clinical code symbols 41C of the clinical code 32. As an example, the function of the code confidence score may be a simple multiplication function, such that the code confidence score is a product of the confidence scores 44 of the plurality of clinical code symbols 41C. Other complex (and weighted) functions may also be contemplated according to the present disclosure.

The instructions 140 further cause the processor 110 to provide the selected grouper code 25 and the selected one or more clinical codes 32 to at least one of an automatic processing application and a user interface. The user interface may be displayed by the output device 160.

The system 100 may automate healthcare revenue cycle while maintaining compliance with the predetermined grouper guidelines 20. Specifically, the system 100 may determine appropriate grouper codes as well as compliant clinical codes (as per the predetermined grouper guidelines 20) based on the electronic medical record 10 without requiring human review. In other words, the system 100 may ensure that billed clinical codes are consistent with billed grouper codes. The system 100 may leverage machine learning technologies to improve a speed and accuracy of billing while reducing human resource time involved in billing.

In some embodiments, the automatic processing application is a billing application. The system 100 may thus enable direct-to-bill (D2B) workflows. For example, the system 100 may enable D2B workflows if the selected one or more clinical codes 32 have the respective code confidence scores that exceed the corresponding confidence score thresholds.

In some embodiments, the selected one or more clinical codes 32 may be provided to the automatic processing application when the confidence score 44 of the grouper code symbol 42G of the selected grouper code 25 and the respective code confidence scores of the selected one or more clinical codes 32 exceed the corresponding confidence score thresholds. Further, the selected one or more clinical codes 32 may be provided to the user interface when the confidence score 44 of the grouper code symbol 42G of the selected grouper code 25 exceeds the corresponding confidence score threshold and the respective code confidence scores of the selected one or more clinical codes 32 do not exceed the corresponding confidence score thresholds.

The user may thus only need to verify the selected one or more clinical codes 32 whose code confidence scores do not exceed the corresponding confidence score thresholds. Therefore, in cases where the code confidence scores of the selected one or more clinical codes 32 do not exceed the corresponding confidence score thresholds, the system 100 may expedite a workflow of the user.

In some embodiments, the instructions 140 may further cause the processor 110 to determine one or more uncertain clinical code symbols 42UC from the plurality of clinical code symbols 41C that have corresponding confidence scores 44 less than a predetermined threshold. Specifically, the processor 110 may determine one or more uncertain clinical code symbols 42UC from the selected one or more clinical codes 32. The instructions 140 may further cause the processor 110 to receive, via the user interface, one or more user inputs to validate the at least one character represented by each of the one or more uncertain clinical code symbols 42UC. This may allow the user to verify the correctness of the one or more uncertain clinical code symbols 42UC, and if incorrect, may allow the user to correct the one or more uncertain clinical code symbols 42UC.

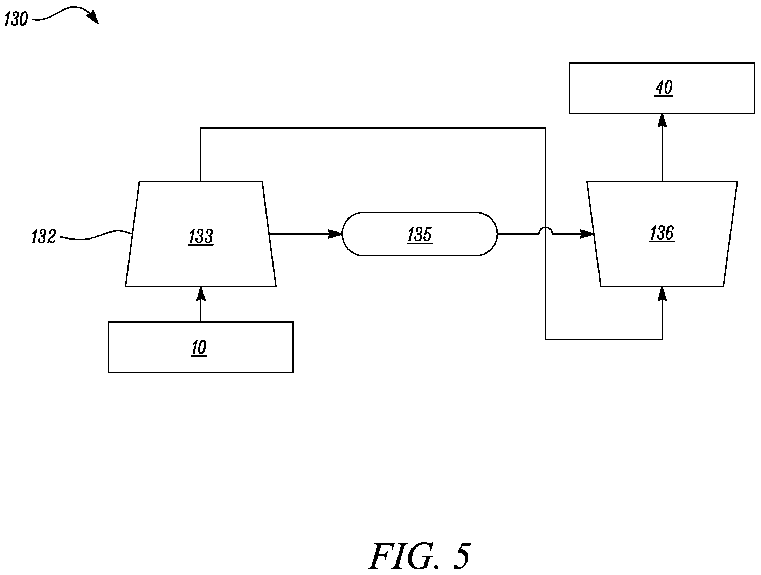

FIG. 5 illustrates a schematic block diagram of the machine learning model 130 according to an embodiment of the present disclosure.

In some embodiments, the machine learning model 130 includes an encoder-decoder architecture. Specifically, in some embodiments, the machine learning model 130 includes an encoder 132 and a decoder 136. The encoder 132 may be configured to receive the electronic medical record 10 and generate a plurality of numerical representations 133 based on the electronic medical record 10. The decoder 136 may be configured to receive the plurality of numerical representations 133 generated by the encoder 132 and generate the output data 40. In some embodiments, the machine learning model 130 may further include an attention module 135 coupling the encoder 132 to the decoder 136. The attention module 135 may be configured to pass a weighted average of the plurality of numerical representations 133 from the encoder 132 to the decoder 136.

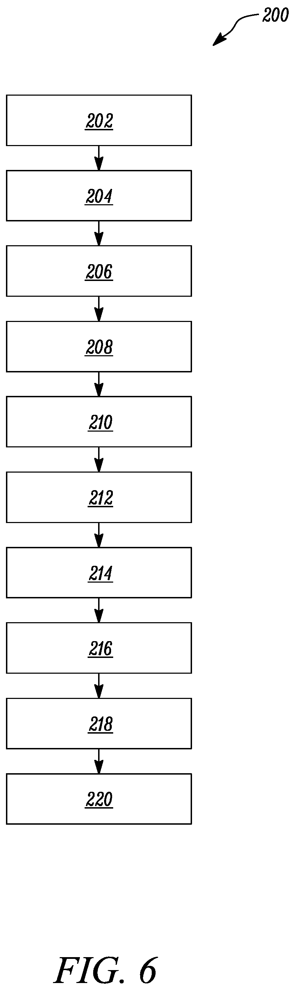

FIG. 6 illustrates a flowchart depicting various steps of a computer-implemented method 200 (hereinafter referred to as "the method 200") for determining healthcare grouper codes with supporting clinical codes concerning an episode of care according to an embodiment of the present disclosure. The method 200 may be implemented, for example, using the system 100 of FIG. 1. The method 200 will be discussed with reference to FIGS. 1-4.

At step 202, the method 200 includes receiving an electronic medical record associated with a patient. For example, the method 200 may include receiving the electronic medical record 10.

At step 204, the method 200 further includes providing the electronic medical record to a machine learning model. The machine learning model is trained on a collection of historical electronic medical records. Each historical electronic medical record is paired with a grouper decision path corresponding to a previously assigned grouper code. For example, the method 200 may include providing the electronic medical record 10 to the machine learning model 130.

At step 206, the method 200 further includes determining, via the machine learning model, a plurality of grouper decision paths based on the electronic medical record and the predetermined grouper guidelines. Each grouper decision path includes a plurality of decision nodes. For example, the method 200 may include determining, via the machine learning model 130, the plurality of grouper decision paths 27 based on the electronic medical record 10 and the predetermined grouper guidelines 20.

At step 208, the method 200 further includes determining, via the machine learning model, a plurality of grouper codes corresponding to the plurality of grouper decision paths. Each grouper code from the plurality of grouper codes is assigned to a corresponding grouper decision path from the plurality of grouper decision paths. For example, the method 200 may include determining, via the machine learning model 130, the plurality of grouper codes 25 corresponding to the plurality of grouper decision paths 27.

At step 210, the method 200 further includes determining, via the machine learning model, a plurality of clinical codes for each grouper decision path, such that each grouper decision path is supported by the respective plurality of clinical codes. Each clinical code from the plurality of clinical codes supports the decision made according to the predetermined grouper guidelines at a corresponding decision node from the plurality of decision nodes of the respective grouper decision path. Each clinical code includes a plurality of characters. For example, the method 200 may include determining, via the machine learning model 130, the plurality of clinical codes 31 for each grouper decision path 28, such that each grouper decision path 28 is supported by the respective plurality of clinical codes 31.

At step 212, the method 200 further includes generating, via the machine learning model, an output data including a plurality of outputs for the plurality of grouper decision paths. Each output for the respective grouper decision path includes a plurality of symbols and a plurality of confidence scores corresponding to the plurality of symbols. Each symbol from the plurality of symbols has a corresponding confidence score from the plurality of confidence scores. The plurality of symbols of each output includes a grouper code symbol representing the grouper code of the respective grouper decision path, such that the plurality of outputs includes a plurality of grouper code symbols having respective confidence scores. The plurality of symbols of each output further includes a plurality of decision symbols representing the decisions made at the plurality of decision nodes of the respective grouper decision path. Each decision symbol from the plurality of decision symbols represents the decision made at the corresponding decision node from the plurality of decision nodes. The plurality of symbols of each output further includes a plurality of clinical code symbols representing the plurality of clinical codes of the respective grouper decision path. Each clinical code symbol from the plurality of clinical code symbols represents at least one character from the plurality of characters of the corresponding clinical code. For example, the method 200 may include generating, via the machine learning model 130, the output data 40.

At step 214, the method 200 further includes selecting one grouper code from the plurality of grouper codes based on the respective confidence scores of the plurality of grouper code symbols. For example, the method 200 may include selecting one grouper code 25 from the plurality of grouper codes 25 based on the respective confidence scores 44 of the plurality of grouper code symbols 42G.

At step 216, the method 200 further includes selecting one or more clinical codes from the plurality of clinical codes of the grouper decision path corresponding to the selected grouper code based on the confidence scores of the plurality of clinical code symbols. Each clinical code from the selected one more clinical codes is at least a partial clinical code. For example, the method 200 may include selecting one or more clinical codes 32 from the plurality of clinical codes 31 of the grouper decision path 28 corresponding to the selected grouper code 25 based on the confidence scores 44 of the plurality of clinical code symbols 41C.

At step 218, the method 200 further includes determining if the confidence score of the grouper code symbol of the selected grouper code and respective code confidence scores of the selected one or more clinical codes exceed the corresponding confidence score thresholds specified by a user. The code confidence score of each clinical code is a function of the confidence scores of the plurality of clinical code symbols of the clinical code. For example, the method 200 may include determining if the confidence score 44 of the grouper code symbol 42G of the selected grouper code 25 and the respective code confidence scores of the selected one or more clinical codes 32 exceed the corresponding confidence score thresholds specified by the user.

At step 220, the method 200 further includes providing the selected grouper code and the selected one or more clinical codes to at least one of an automatic processing application and a user interface. For example, the method 200 may include providing the selected grouper code 25 and the selected one or more clinical codes 32 to at least one of the automatic processing application and the user interface.

The method 200 may automate healthcare revenue cycle while maintaining compliance with the predetermined grouper guidelines 20. Specifically, the method 200 may be used to determine appropriate grouper codes as well as compliant clinical codes (as per the predetermined grouper guidelines 20) based on the electronic medical record 10 without requiring human review. In other words, the method 200 may ensure that billed clinical codes are consistent with billed grouper codes. The method 200 may leverage machine learning technologies to improve a speed and accuracy of billing while reducing human resource time involved in billing.

In some embodiments, the automatic processing application is a billing application. The method 200 may thus enable direct-to-bill (D2B) workflows. For example, the method 200 may enable D2B workflows if the selected one or more clinical codes 32 have the respective code confidence scores that exceed the corresponding confidence score thresholds.

In some embodiments, the selected one or more clinical codes are provided to the automatic processing application when the confidence score of the grouper code symbol of the selected grouper code and the respective code confidence scores of the selected one or more clinical codes exceed the corresponding confidence score thresholds and provided to the user interface when the confidence score of the grouper code symbol of the selected grouper code exceeds the corresponding confidence score threshold and the respective code confidence scores of the selected one or more clinical codes do not exceed the corresponding confidence score thresholds.

For example, the selected one or more clinical codes 32 may be provided to the automatic processing application when the confidence score 44 of the grouper code symbol 42G of the selected grouper code 25 and the respective code confidence scores of the selected one or more clinical codes 32 exceed the corresponding confidence score thresholds. Further, the selected one or more clinical codes 32 may be provided to the user interface when the confidence score 44 of the grouper code symbol 42G of the selected grouper code 25 exceeds the corresponding confidence score threshold and the respective code confidence scores of the selected one or more clinical codes 32 do not exceed the corresponding confidence score thresholds.

The user may thus only need to verify the selected one or more clinical codes 32 whose code confidence scores do not exceed the corresponding confidence score thresholds. Therefore, in cases where the code confidence scores of the selected one or more clinical codes 32 do not exceed the corresponding confidence score thresholds, the method 200 may expedite a workflow of the user.

In some embodiments, for each decision node of the respective grouper decision path, the corresponding output includes a set of clinical code symbols arranged in a code sequence to together form the clinical code assigned to the decision made at the decision node. For example, for each decision node 22 of the respective grouper decision path 28, the corresponding output 51 may include the set of clinical code symbols 41C arranged in the code sequence to together form the clinical code 32 assigned to the decision made at the decision node 22.

In some embodiments, the plurality of decision nodes is arranged in a node sequence in each grouper decision path. The plurality of decision symbols and the plurality of clinical code symbols are together arranged in an output sequence that corresponds to the node sequence of the plurality of decision nodes. For example, the plurality of decision nodes 21 may be arranged in the node sequence in each grouper decision path 28, and the plurality of decision symbols 41D and the plurality of clinical code symbols 41C may be together arranged in the output sequence that corresponds to the node sequence of the plurality of decision nodes 21.

In some embodiments, for each decision node with an assigned clinical code, the corresponding one or more decision symbols are followed by the corresponding one or more clinical code symbols in the output sequence. For example, for each decision node 22 with an assigned clinical code 32, the corresponding one or more decision symbols 42D may be followed by the corresponding one or more clinical code symbols 42C in the output sequence.

In some embodiments, for each decision node of the respective grouper decision path without an assigned clinical code, the corresponding output is devoid of any clinical code symbol corresponding to the decision node. For example, for each decision node 22 of the respective grouper decision path 28 without an assigned clinical code, the corresponding output 51 may be devoid of any clinical code symbol corresponding to the decision node 22.

In some embodiments, the grouper code symbol is arranged at a beginning or an end of the output sequence. For example, the grouper code symbol 42G may be arranged at a beginning or an end of the output sequence.

In some embodiments, each output includes a vector including the plurality of symbols. For example, each output 51 may include the vector 55 including the plurality of symbols 41.

In some embodiments, the method 200 further includes determining one or more uncertain clinical code symbols from the plurality of clinical code symbols that have corresponding confidence scores less than a predetermined threshold. In some embodiments, the method 200 further includes receiving, via the user interface, one or more user inputs to validate the at least one character represented by each of the one or more uncertain clinical code symbols. For example, the method 200 may include determining the one or more uncertain clinical code symbols 42UC from the plurality of clinical code symbols 41C that have corresponding confidence scores 44 less than the predetermined threshold, and receiving, via the user interface, the one or more user inputs to validate the at least one character represented by each of the one or more uncertain clinical code symbols 42UC. This may allow the user to verify the correctness of the one or more uncertain clinical code symbols 42UC, and if incorrect, may allow the user to correct the one or more uncertain clinical code symbols 42UC.

It should be appreciated by one of ordinary skill in the art that the systems and techniques described herein are necessarily rooted in computing technology for automatically generating grouper output and compliance. For instance, coding guidelines are subject to different interpretations by organizations and medical coders, which creates substantial variability in actual medical coding practices. The subject matter disclosed herein provides a means to estimate grouper output and its compliance (aspects/symbols of medical codes relevant to grouper logic) without fully determining all the medical codes for an EMR whose choice, presence/absence, or level of specificity may vary depending on the manner in which the coding system is configured, which may take into account coding practices found in different organizations or practices by individual medical coders. By reducing sensitivity of the modeling computation (by virtue of the different interpretations across organizations and medical coders), the subject matter disclosed herein results in superior accuracy in automatically determining grouper output and compliance, compared to industry standards.

Relatedly, the subject matter disclosed herein also provides capabilities superior to rule-based technologies. First and foremost, such rule-based technologies to solve the technical problems disclosed herein do not exist. But even if developed, such rule-based approaches may not achieve comparable accuracy to the machine learning based approaches of the instant disclosure for at least the reason that such rule-based approaches could not account for differences between coding practices found in different organizations without substantial manual configurations. Stated differently, the instant disclosure outperforms traditional rule-based approaches because it can adapt to organizational practices without relying on manual configuration steps. This, in turn, can improve the overall accuracy of the system over-time because manual configurations can introduce errors in the output generated by such rules.

Additionally, it should be understood that the present disclosure improves the underlying system in a manner that is impracticable for a human to perform, with or without the aid of pen and paper. For instance, for the present disclosure to provide accuracy improvements, clinical documentation practices across different institutions in data corpora that would be too voluminous for manual analysis must be analyzed to develop the relevant models. For instance, systems of the instant disclosure process hundreds of millions of documents, or more, to adequately train the underlying machine learning models. Furthermore, as it should be appreciated by one of ordinary skill in the art, the relevant computations disclosed herein are advantageously computed at nearly real-time speed to meet the time constraints of healthcare business operations. For instance, the act of training the machine learning model may involve forward and backpropagation each involving millions of discrete computations per concept and/or grouper code. As a result, a system utilizing such a model to automatically generate grouper output would require hundreds of millions of discrete calculations to make operable, resulting in computational complexity that would prevent a human from being able to perform the techniques of the present disclosure.

Unless otherwise indicated, all numbers expressing feature sizes, amounts, and physical properties used in the specification and claims are to be understood as being modified by the term "about." Accordingly, unless indicated to the contrary, the numerical parameters set forth in the foregoing specification and attached claims are approximations that can vary depending upon the desired properties sought to be obtained by those skilled in the art utilizing the teachings disclosed herein.

Although specific embodiments have been illustrated and described herein, it will be appreciated by those of ordinary skill in the art that a variety of alternate and/or equivalent implementations can be substituted for the specific embodiments shown and described without departing from the scope of the present disclosure. This application is intended to cover any adaptations or variations of the specific embodiments discussed herein. Therefore, it is intended that this disclosure be limited only by the claims and the equivalents thereof.

## Claims

1. A system for determining healthcare grouper codes with supporting clinical codes concerning an episode of care, the system comprising:
one or more computer processors; and
at least one non-transitory computer-readable storage, communicatively coupled to the one or more computer processors, having stored thereon a machine learning model and instructions that when executed by the one or more computer processors cause the one or more computer processors to:
receive an electronic medical record associated with a patient;
provide the electronic medical record to the machine learning model, wherein the machine learning model is trained on a collection of historical electronic medical records, wherein each historical electronic medical record is paired with a grouper decision path corresponding to a previously assigned grouper code;
determine, via the machine learning model, a plurality of grouper decision paths based on the electronic medical record and predetermined grouper guidelines, wherein each grouper decision path comprises a plurality of decision nodes;
determine, via the machine learning model, a plurality of grouper codes corresponding to the plurality of grouper decision paths, wherein each grouper code from the plurality of grouper codes is assigned to a corresponding grouper decision path from the plurality of grouper decision paths;
determine, via the machine learning model, a plurality of clinical codes for each grouper decision path, such that each grouper decision path is supported by the respective plurality of clinical codes, wherein each clinical code from the plurality of clinical codes supports the decision made according to the predetermined grouper guidelines at a corresponding decision node from the plurality of decision nodes of the respective grouper decision path, and wherein each clinical code comprises a plurality of characters;
generate, via the machine learning model, an output data comprising a plurality of outputs for the plurality of grouper decision paths, wherein each output for the respective grouper decision path comprises a plurality of symbols and a plurality of confidence scores corresponding to the plurality of symbols, wherein each symbol from the plurality of symbols has a corresponding confidence score from the plurality of confidence scores, the plurality of symbols of each output comprising:
a grouper code symbol representing the grouper code of the respective grouper decision path, such that the plurality of outputs comprises a plurality of grouper code symbols having respective confidence scores;
a plurality of decision symbols representing the decisions made at the plurality of decision nodes of the respective grouper decision path, wherein each decision symbol from the plurality of decision symbols represents the decision made at the corresponding decision node from the plurality of decision nodes; and
a plurality of clinical code symbols representing the plurality of clinical codes of the respective grouper decision path, wherein each clinical code symbol from the plurality of clinical code symbols represents at least one character from the plurality of characters of the corresponding clinical code;
select one grouper code from the plurality of grouper codes based on the respective confidence scores of the plurality of grouper code symbols;
select one or more clinical codes from the plurality of clinical codes of the grouper decision path corresponding to the selected grouper code based on the confidence scores of the plurality of clinical code symbols, wherein each clinical code from the selected one more clinical codes is at least a partial clinical code;
determine if the confidence score of the grouper code symbol of the selected grouper code and respective code confidence scores of the selected one or more clinical codes exceed corresponding confidence score thresholds specified by a user, wherein the code confidence score of each clinical code is a function of the confidence scores of the plurality of clinical code symbols of the clinical code; and
provide the selected grouper code and the selected one or more clinical codes to at least one of an automatic processing application and a user interface.

2. The system of claim 1, wherein the selected one or more clinical codes are provided to the automatic processing application when the confidence score of the grouper code symbol of the selected grouper code and the respective code confidence scores of the selected one or more clinical codes exceed the corresponding confidence score thresholds and provided to the user interface when the confidence score of the grouper code symbol of the selected grouper code exceeds the corresponding confidence score threshold and the respective code confidence scores of the selected one or more clinical codes do not exceed the corresponding confidence score thresholds.

3. The system of any of claims 1-2, wherein, for each decision node of the respective grouper decision path, the corresponding output comprises a set of clinical code symbols arranged in a code sequence to together form the clinical code assigned to the decision made at the decision node.

4. The system of any of claims 1-3, wherein the plurality of decision nodes is arranged in a node sequence in each grouper decision path, and wherein the plurality of decision symbols and the plurality of clinical code symbols are together arranged in an output sequence that corresponds to the node sequence of the plurality of decision nodes.

5. The system of any of claims 1-4, wherein, for each decision node with an assigned clinical code, the corresponding one or more decision symbols are followed by the corresponding one or more clinical code symbols in the output sequence.

6. The system of any of claims 1-5, wherein the grouper code symbol is arranged at a beginning or an end of the output sequence.

7. The system of any of claims 1-6, wherein, for each decision node of the respective grouper decision path without an assigned clinical code, the corresponding output is devoid of any clinical code symbol corresponding to the decision node.

8. The system of any of claims 1-7, wherein each output comprises a vector comprising the plurality of symbols.

9. The system of any of claims 1-8, wherein the one or more processors are further configured to:
determine one or more uncertain clinical code symbols from the plurality of clinical code symbols that have corresponding confidence scores less than a predetermined threshold; and
receive, via the user interface, one or more user inputs to validate the at least one character represented by each of the one or more uncertain clinical code symbols.

10. The system of any of claims 1-9, wherein the machine learning model comprises:
an encoder configured to receive the electronic medical record and generate a plurality of numerical representations based on the electronic medical record; and
a decoder configured to receive the plurality of numerical representations generated by the encoder and generate the output data.

11. The system of any of claims 1-10, wherein the machine learning model further comprises an attention module coupling the encoder to the decoder, and wherein the attention module is configured to pass a weighted average of the plurality of numerical representations from the encoder to the decoder.

12. The system of any of claims 1-11, wherein the automatic processing application is a billing application.

13. The system of any of claims 1-12, wherein the predetermined grouper guidelines correspond to at least one of a diagnosis-related group (DRG) and an enhanced ambulatory patient group (EAPG).
